# EUROPEAN PATENT SPECIFICATION

(11) **EP 1 637 153 B1**
(45) Date of publication and mention of the grant of the patent: **04.03.2009**
(21) Application number: 05108466.3
(22) Date of filing: 15.09.2005
(51) Int. Cl.: A61K 36/324, A61K 36/539, A61P 29/00

(54) **Product with selective antinflammatory action and antifibrotic properties**
Produkt mit selektiver entzündungshemmender Wirkung und antifibrotischen Eigenschaften
Produit avec activité anti-inflammatoire selective et propriétés antifibrotiques

(30) Priority: 16.09.2004 IT RM20040439
(43) Date of publication of application: 22.03.2006
(73) Proprietor: Ca. Di. Group S.r.l., 00124 Roma (IT)
(72) Inventor: CARAMELLI, Gianfranco, 00124, Roma (IT)
(74) Representative: Germinario, Claudio

(56) References cited:
- WO-A-20/05002611
- US-A1- 2003 199 581
- US-B1- 6 224 871
- US-B1- 6 492 429

## Description

The chronic non-infectious inflammatory diseases represent the most frequent illness and are progressively increasing in the Western industrialised countries. They can affect several organs including heart, large vessels, kidneys, lungs, skin, articulations, liver, pancreas, intestine, etc. The chronic inflammation in the majority of cases starts a process of tissue fibrogenesis that can cause important tissue remodelling with functional alterations, sometimes severe, of the affected organs.

The anti-inflammatory drugs actually used in the clinical practice are effective to control the inflammatory process in the majority of the pathological diseases, but do not interrupt the development of the tissue fibrosis and therefore the functional deterioration of the affected organ. Consequently such diseases are in the majority of cases inexorably progressive. Furthermore, the majority of ant inflammatory drugs currently used are burdened with frequent and often severe adverse undesirable events. Therefore in present clinical practice, pharmaceutical products having at the same time both anti-inflammatory and antifibrotic action are urgently needed.

The chronic inflammation and the tissue fibrogenesis are two interconnected processes, regulated by specific inflammation mediators the most important of which are oxygen radicals, cytokines and arachidonic acid metabolites. The oxygen radicals are almost all proinflammatory and profibrogenic. Cytokines are distinguished in several classes: proinflammatory, antiinflammatory, profibrotic and antifibrotic cytokines. The metabolic products from arachidonic acid are constituted by two main classes: the prostaglandins with protective action and the leucotrienes with proinflammatory effects, produced by the activation of cycloxigenase and lypoxigenase, respectively.

The anti-inflammatory drugs currently used in clinical practice mainly act by the inhibition of the production and release of proinflammatory citokines and by the inhibition, at the same time, of both the cycloxigenase and lipoxigenase. Therefore, although they reduce the proinflammatory mediator production, at the same time they reduce the prostaglandins with protective action. This conflicting mechanism of action would be responsible for the frequent adverse events of the currently used anti-inflammatory drugs. These drugs do not affect the fibrogenetic mediators.

Therefore in the state of the art there was the need for a product that, apart from showing antiinflammatory action without the typical adverse events of the products known in the art, could offer also antifibrotic action, in particular anti-inflammatory and specific antifibrotic action for specific organs.

For all these reasons the applicant has planned to develop a new product that had selective anti-inflammatory activity, in particular inhibiting the lipoxigenase and the production of proinflammatory cytokines, without inhibiting the production of the protective prostaglandins, and at the same time reduced the tissue fibrogenesis secondary to chronic inflammation. Such product is made of an association of an extract of Boswellia Serrata, with high content in boswellic acids, and of a plant extract with antifibrotic action. The applicant of the present invention discovered that extracts of plant origin can be advantageously used in order to obtain an antifibrotic action and can be joined to Boswellia anti-inflammatory extract; the choice of extracts from special plants, reported in the following, allows to obtain specific action for separate organs like: liver, intestine, lungs, vascular system. Both Boswellia and the studied antifibrotics being significant adverse events free, their association can be given also for long periods and in a repeatable way.

Therefore it is subject of the present invention the use of an association of Boswellia Serrata extract with a plant antifibrotic agent coming from one or more of the following species
- Astragalus Membranaceus
- Astragalus Poligonum
- Angelica Sinensis
- Camellia Sinensis
- Salvia Miltiorrhizae
- Scutellaria Baicalensis
- Ginkgo Biloba
- Polygonum Aviculare
- Gynostemma Pentaphyllum
- Picrorhiza Kurroa
for the production of a medicament, against inflammatory and fibrotic states.

The association contains Boswellia and antifibrotic agent in percent comprised between 5-95% and 50-50% in weight with reference to dry extracts. Advantageously the association is given in order to guarantee the administration of quantities of Boswellia, as dry extract, between 300 mg and 1200 mg/die.

Further subject of the invention are compositions that contain the above association, for use in the treatment of inflammatory and fibrotic courses, formulated as capsules or tablets possibly with added excipients for producing pills, or other administrable formulation.

Another subject of the present invention is a dietary integrator useful in helping the treatment of flogistic status and fibrogenesis, containing the association between a dry extract of Boswellia Serrata and dry extract of a plant antifibrotic agent coming from one or more of the following species
- Astragalus Membranaceus
- Astragalus Poligonum
- Angelica Sinensis
- Camellia Sinensis
- Salvia Miltiorrhizae
- Scutellaria Baicalensis
- Ginkgo Biloba
- Polygonum Aviculare
- Gynostemma Pentaphyllum
- Picrorhiza Kurroa
in percent comprised between 5-95% and 50-50% in weight related to dry extracts.

Another subject of the present invention is an association of a Boswellia Serrata extract and a plant antifibrotic agent as a medicament, wherein said plant antifibrotic agent comes from one or more of the following species:
- Astragalus Membranaceus
- Astragalus Poligonum
- Angelica Sinensis
- Salvia Miltiorrhizae
- Polygonum Aviculare
- Gynostemma Pentaphyllum
It is very important to put in evidence, as regards the nature and characteristics of chosen components, the following information:

### BOSWELLIA SERRATA EXTRACTS

From this plant is extracted a gummy oleoresin containing oils, terpenoids and gums. From a therapeutic point of view, the terpenoids are the most important active components, extensively studied from a long time. Among the terpenoids, 4 triterpenic pentacyclic acids, named boswellic acids, were identified which result to be responsible for the antinflammatory and antiarthritic action of the Boswellia Serrata extracts.

Such actions are carried on through the inhibition of the 5-lipoxigenase (5-LO) enzyme so preventing the production of pro-inflammatory leucotrienes. Normally the other antinflammatory agents act, on the contrary, at level of both cycloxigenase enzymes 1 and 2. Related to osteo-arthritic disease, it seems that boswellic acids also inhibit the enzymes responsible for glycosaminoglycans degradation including beta-glucuronidase, beta-N-acetilglycosaminidase-catepsine B₁, B₂, and D and the human leukocyte elastase (HLE) a serine proteasis that starts the degradation of the joint tissues.

This double mechanism of action of the boswellic acids, simultaneous inhibition of the 5-LO and HLE, is unique among anti-inflammatory agents, as reported by recent studies. This mechanism is in the extreme important since, from "in vitro" observations, it was supposed that E.L.A., produced and released from polimorphonucleatics leucocytes (PMNLₛ) plays an important role as damaging agent in many serious pathologies characterised from a high degree of structures deterioration, like lung emphysema, the chronic bronchitis, the glomerulonephritis and rheumatoid arthritis.

Other compounds that are able to interfere with the 5-LO activity like cortisol, testosteron, 18-β-glycyrrhetinic acid did not show the same action on the HLE activity. Therefore this double mechanism of enzymatic inhibition at joint level is proven only for the boswellic acids.

Moreover recent studies evaluating boswellic acids and antioxidant agents have shown that a product with anti-inflammatory properties is also an antioxidant and that the antioxidant mechanism is also responsible for the anti-inflammatory action.

These actions, comparable to those of the non-steroidal anti-inflammatory drugs (NSAID), are obtained without significant adverse events, neither in acute nor in chronic treatments, so that the FDA has approved the use of Boswellia Serrata extract also for alimentary products.

In the present invention, as Boswellia is used only the acid fraction of the extract with 80% of boswellic acids, preferably with 90% and, more preferably with about 95% of boswellic acids. The dry extract used is obtained following methods known in the art. The boswellic acids are 4 pentacyclic triterpenic acids: β-boswellic acid, acetyl-β-boswellic acid, 11-keto-β-boswellic acid and acetyl-11-keto-β-boswellic acid. The more abundant of the mixture is the β-boswellic acid.

With regard to Boswellia dry extract, this is obtained following well known technical methodologies, starting from its gummy oleoresin utilising only the acid fraction of the extract. In the present invention this acid part will be indicated as acid fraction.

### PLANT ANTIFIBROTIC AGENT

The inflammatory chronic diseases, that can involve several organs, start a tissue fibrogenetic process that may induce functional alterations, often severe, of the affected organs. The applicant discovered that a few plants, well known for having various actions, have shown a good antifibrotic effect, through different mechanisms, inhibiting or at least reducing the fibrogenesis.

According to these known mechanisms, the activation process of the Kupffer cells is hampered, the hepatic enzyme decrease, the production and content of collagene is reduced, its composition is improved, the fibroblast production is inhibited, the modulation of cellular TGF-β₁/Smads pathway is obtained. Such effects that may also be maintained after the interruption of the treatment. The applicant discovered that antifibrotic action of those plant extracts can be usefully combined with the anti-inflammatory action of Boswellia in order to obtain a synergic and specific effect on one or more organs. Therefore using the properties of various plant products with antifibrotic action it will be possible to choose the most specific and suitable action for the various fibrogenetic diseases of any single patient.

For this reason the proposed association will have always the Boswellia Serrata as anti-inflammatory agent, whereas the antifibrotic agent to be combined could be chosen with regard to the organ involved by a fibrogenetic process, among the extract of one or more of the following plants:
- Astragalus Membranaceus
- Astragalus Poligonum
- Angelica Sinensis
- Camellia Sinensis
- Salvia Miltiorrhizae
- Scutellaria Baicalensis
- Ginkgo Biloba
- Polygonum Aviculare
- Gynostemma Pentaphyllum
- Picrorhiza Kurroa

It is to be noted that the plant products referred to show no side effect. In the meaning of the present invention as plant antifibrotic agent it is used a dry extract from one or more plants previously reported; the respective extracts are obtained with well known technical methods. Preferably the chosen Scutellaria has a 90-95% Baicalin contents (its most active flavone).

Furthermore it was discovered and this is a further subject of the present invention that an association of Boswellia Serrata and Scutellaria Baicalensis shows a particular antifibrotic and antinflammatory effectiveness against liver and intestine fibrosis and inflammation.

The compositions consist of dry extracts, in the previously indicated range of percentages, in capsules or, possibly with added safe excipients, in tablets, powder to be dissolved in water or other well known and used administration ways.
It is reported as not restrictive example the composition of the contents of capsules used for the treatment of liver and intestine fibrosis and inflammation.

| | |
|---|---|
| Boswellia Serrata dry extract (acid fraction) | 600 mg |
| Scutellaria Baicalensis dry extract | 1500 mg |

## Claims

1. Use of an association of a Boswellia Serrata extract and a plant antifibrotic agent for the production of a medicament against inflammatory and fibrotic courses, wherein said plant antifibrotic agent comes from one or more of the following species:
• Astragalus Membranaceus
• Astragalus Poligonum
• Angelica Sinensis
• Camellia Sinensis
• Salvia Miltiorrhizae
• Scutellaria Baicalensis
• Ginkgo Biloba
• Polygonum Aviculare
• Gynostemma Pentaphyllum
• Picrorhiza Kurroa

2. Use according to claim 1, wherein Boswellia Serrata is present in quantity from 5 to 50% and said plant antifibrotic agent in quantity from 50 to 95% by weight, preferably Boswellia in quantity of 25-50% and said plant antifibrotic agent in quantity of 50-75%.

3. Use according at least to one of the previous claims, wherein Boswellia Serrata and said plant antifibrotic agent are both in quantity of 50% by weight.

4. Use according at least to one of the previous claims, wherein Boswellia Serrata is present as a dry extract of its acid fraction with a content of boswellic acids of 90-95% and said antifibrotic agent as dry extract.

5. Use according at least to one of the previous claims, wherein said plant antifibrotic agent is made of a Scutellaria Baicalensis extract.

6. Use according to claim 5 for the production of a medicament against intestinal and liver inflammations.

7. Use according to claim 6, wherein Boswellia Serrata is present as dry extract of the acid fraction in quantity from 5 to 50% and said plant antifibrotic agent as dry extract in quantity from 50 to 95% in weight.

8. Use according at least to one of the claims from 5 to 7, wherein said plant antifibrotic agent shows a content of 90-95% of Baicalin.

9. Compositions containing an association of Boswellia Serrata extract and a plant antifibrotic agent from one or more of the plant species indicated in claim 1 together with allowed and safe excipients, as capsules, tablets and other administrable forms for use in the treatment of inflammatory and fibrotic courses.

10. Dietary supplement, as coadjuvant for use in the treatment of inflammatory and fibrotic courses, containing an association as claimed in claims from 1 to 8.

11. Association of a Boswellia Serrata extract and a plant antifibrotic agent as a medicament, wherein said plant antifibrotic agent comes from one or more of the following species:
• Astragalus Membranaceus
• Astragalus Poligonum
• Angelica Sinensis
• Salvia Miltiorrhizae
• Polygonum Aviculare
• Gynostemma Pentaphyllum

12. Association of a Boswellia Serrata extract and a plant antifibrotic agent according to claim 11 as a medicament against inflammatory and fibrotic courses.

13. Association according to one of the claims 11 or 12, wherein Boswellia Serrata is present in quantity from 5 to 50% and said plant antifibrotic agent in quantity from 50 to 95% by weight, preferably Boswellia in quantity of 25-50% and said plant antifibrotic agent in quantity of 50-75%.

14. Association according to one of the claims from 11 to 13, wherein Boswellia Serrata and said plant antifibrotic agent are both in quantity of 50% by weight.

15. Association according to one of the claims from 11 to 14, wherein Boswellia Serrata is present as a dry extract of its acid fraction with a content of boswellic acids of 90-95% and said antifibrotic agent as dry extract.

## Patentansprüche

1. Verwendung einer Kombination von einem Boswellia Serrata-Extrakt und einem pflanzlichen antifibrotischen Agens für die Herstellung eines Medikaments gegen entzündliche und fibrotische Verläufe, wobei das pflanzliche antifibrotische Agens aus einer oder mehreren der folgenden Spezies stammt:
• Astragalus Membranaceus
• Astragalus Poligonum
• Angelica Sinensis
• Camellia Sinensis
• Salvia Miltiorrhizae
• Scutellaria Baicalensis
• Ginkgo Biloba
• Polygonum Aviculare
• Gynostemma Pentaphyllum
• Picrorhiza Kurroa

2. Verwendung nach Anspruch 1, wobei Boswellia Serrata in einer Menge von 5 bis 50 % und das pflanzliche antifibrotische Agens in einer Menge von 50 bis 95 Gew.-%, vorzugsweise Boswellia in einer Menge von 25-50 % und das pflanzliche antifibrotische Agens in einer Menge von 50-75 % vorhanden ist.

3. Verwendung nach wenigstens einem der vorangehenden Ansprüche, wobei Boswellia Serrata und das pflanzliche antifibrotische Agens beide in einer Menge von 50 Gew.-% vorliegen.

4. Verwendung nach wenigstens einem der vorangehenden Ansprüche, wobei Boswellia Serrata als ein Trockenextrakt seiner Säurefraktion mit einem Gehalt von Boswellia-Säuren von 90-95 % und das antifibrotische Agens als Trockenextrakt vorhanden ist.

5. Verwendung nach wenigstens einem der vorangehenden Ansprüche, wobei das pflanzliche antifibrotische Agens aus einem Scutellaria Baicalensis-Extrakt hergestellt ist.

6. Verwendung nach Anspruch 5 für die Herstellung eines Medikaments gegen Darm- und Leberentzündungen.

7. Verwendung nach Anspruch 6, wobei Boswellia Serrata als Trockenextrakt der Säurefraktion in einer Menge von 5 bis 50 % und das pflanzliche antifibrotische Agens als Trockenextrakt in einer Menge von 50 bis 95 Gew.-% vorhanden ist.

8. Verwendung nach wenigstens einem der Ansprüche 5 bis 7, wobei das pflanzliche antifibrotische Agens einen Gehalt von 90-95 % Baicalin aufweist.

9. Zusammensetzungen, enthaltend eine Kombination von Boswellia Serrata-Extrakt und einem pflanzlichen antifibrotischen Agens aus einer oder mehreren der in Anspruch 1 angegebenen Pflanzenspezies zusammen mit zugelassenen und sicheren Exzipienten, als Kapseln, Tabletten und andere verabreichbare Formen zur Verwendung bei der Behandlung von entzündlichen und fibrotischen Verläufen.

10. Nahrungsergänzungsmittel, als Coadjuvans zur Verwendung bei der Behandlung von entzündlichen und fibrotischen Verläufen, enthaltend eine Kombination wie in den Ansprüchen 1 bis 8 beansprucht.

11. Kombination von einem Boswellia Serrata-Extrakt und einem pflanzlichen antifibrotischen Agens als ein Medikament, wobei das pflanzliche antifibrotische Agens aus einer oder mehreren der folgenden Spezies stammt:
• Astragalus Membranaceus
• Astragalus Poligonum
• Angelica Sinensis
• Salvia Miltiorrhizae
• Polygonum Aviculare
• Gynostemma Pentaphyllum

12. Kombination von einem Boswellia Serrata-Extrakt und einem pflanzlichen antifibrotischen Agens nach Anspruch 11, als ein Medikament gegen entzündliche und fibrotische Verläufe.

13. Kombination nach einem der Ansprüche 11 oder 12, wobei Boswellia Serrata in einer Menge von 5 bis 50 % und das pflanzliche antifibrotische Agens in einer Menge von 5 bis 95 Gew.-%, vorzugsweise Boswellia in einer Menge von 25-50 % und das pflanzliche antifibrotische Agens in einer Menge von 50-75 % vorhanden ist.

14. Kombination nach einem der Ansprüche 11 bis 13, wobei Boswellia Serrata und das pflanzliche antifibrotische Agens beide in einer Menge von 50 Gew.-% vorliegen.

15. Kombination nach einem der Ansprüche 11 bis 14, wobei Boswellia Serrata als ein Trockenextrakt seiner Säurefraktion mit einem Gehalt von Boswellia-Säuren von 90-95 % und das antifibrotische Agens als Trockenextrakt vorhanden ist.

## Revendications

1. Utilisation d'une association d'un extrait de Boswellia Serrata et d'un agent végétal antifibrotique pour la production d'un médicament luttant contre les évolutions inflammatoires et fibrotiques, dans laquelle ledit agent végétal antifibrotique provient d'une ou de plusieurs des espèces suivantes :
- Astragalus Membranaceus
- Astragalus Poligonum
- Angelica Sinensis
- Camellia Sinensis
- Salvia Miltiorrhizae
- Scutellaria Baicalensis
- Ginkgo Biloba
- Polygonum Aviculare
- Gynostemma Pentaphyllum
- Picrorhiza Kurroa

2. Utilisation selon la revendication 1, dans laquelle Boswellia Serrata est présente en une quantité de 5 à 50 % et ledit agent végétal antifibrotique est présent en une quantité de 50 à 95 % en poids, de préférence Boswellia en une quantité de 25 à 50 % et ledit agent végétal antifibrotique en une quantité de 50 à 75 %.

3. Utilisation selon au moins une des revendications précédentes, dans laquelle Boswellia Serrata et ledit agent végétal antifibrotique sont tous les deux présents en une quantité de 50 % en poids.

4. Utilisation selon au moins une des revendications précédentes, dans laquelle Boswellia Serrata est présente sous forme d'un extrait sec de sa fraction acide avec une teneur en acides boswelliques de 90 à 95 % et ledit agent antifibrotique est présent sous forme d'extrait sec.

5. Utilisation selon au moins une des revendications précédentes, dans laquelle ledit agent végétal antifibrotique est fait d'un extrait de Scutellaria Baicalensis.

6. Utilisation selon la revendication 5, pour la production d'un médicament luttant contre les inflammations intestinales et hépatiques.

7. Utilisation selon la revendication 6, dans laquelle Boswellia Serrata est présente sous la forme d'extrait sec de la fraction acide en une quantité de 5 à 50 % et ledit agent végétal antifibrotique est présent sous la forme d'extrait sec en une quantité de 50 à 95 % en poids.

8. Utilisation selon au moins une des revendications 5 à 7, dans laquelle ledit agent végétal antifibrotique présente une teneur en Baicaline de 90 à 95 %.

9. Compositions contenant une association d'extrait de Boswellia Serrata et d'un agent végétal antifibrotique provenant d'une ou de plusieurs des espèces végétales indiquées dans la revendication 1, associés à des excipients autorisés et sans dangers, sous forme de gélules, comprimés ou autres formes pouvant être administrées pour une utilisation dans le traitement des évolutions inflammatoires et fibrotiques.

10. Supplément diététique, en tant que coadjuvant pour une utilisation dans le traitement des évolutions inflammatoires et fibrotiques, contenant une association telle que revendiquée dans les revendications 1 à 8.

11. Association d'un extrait de Boswellia Serrata et d'un agent végétal antifibrotique sous forme d'un médicament, dans laquelle ledit agent végétal antifibrotique provient d'une ou de plusieurs des espèces suivantes :
- Astragalus Membranaceus
- Astragalus Poligonum
- Angelica Sinensis
- Salvia Miltiorrhizae
- Polygonum Aviculare
- Gynostemma Pentaphyllum

12. Association d'un extrait de Boswellia Serrata et d'un agent végétal antifibrotique selon la revendication 11, sous forme d'un médicament luttant contre les évolutions inflammatoires et fibrotiques.

13. Association selon l'une des revendications 11 ou 12, dans laquelle Boswellia Serrata est présente en une quantité de 5 à 50 % et ledit agent végétal antifibrotique est présent en une quantité de 50 à 95 % en poids, de préférence Boswellia en une quantité de 25 à 50 % et ledit agent végétal antifibrotique en une quantité de 50 à 75%.

14. Association selon l'une des revendications 11 à 13, dans laquelle Boswellia Serrata et ledit agent végétal antifibrotique sont tous les deux présents en une quantité de 50 % en poids.

15. Association selon l'une des revendications 11 à 14, dans laquelle Boswellia Serrata est présente sous la forme d'un extrait sec de sa fraction acide avec une teneur en acides boswelliques de 90 à 95 % et ledit agent antifibrotique est présent sous la forme d'extrait sec.
